# EUROPEAN PATENT APPLICATION

(11) **EP 1 536 229 A1**
(43) Date of publication of application: **01.06.2005**
(21) Application number: 03078775.8
(22) Date of filing: 26.11.2003
(51) Int. Cl.: G01N 33/50, C12Q 1/02

(54) **Method to identify a biomolecular profile**

(71) Applicant: Plant Research International B.V., 6708 PB Wageningen (NL)
(72) Inventor: Bosch, Hendrik Jan, 6708 NA Wageningen (NL); van der Krol, Alexander Ronald, 3511 MN Utrecht (NL)
(74) Representative: Prins, Adrianus Willem, Mr. Ir.

(57) **Abstract**

This invention relates to identifying a biomolecular profile of an organism. In particular, the invention relates to targeted and selected proteomics. Provided is a method for identifying a molecular profile comprising a set of molecules of an organism, said method comprising the steps of:
a) providing an organism with at least one heterologous modifying activity capable of tagging at least part of said molecules with at least one tag, preferably a carbohydrate tag, which is otherwise not displayed in said organism, wherein said tagging is regulated by at least one regulatory element; b) isolating the tagged molecules to obtain a library of tagged molecules; and c) characterizing said tagged molecules.

## Description

This invention relates to identifying a biomolecular, or molecular, profile of an organism. In particular, the invention relates to targeted and selected proteomics.

Over the past decade, molecular profiling has emerged as a dynamic new discipline, capable of generating a global view of both quantitative and qualitative aspects of proteins, lipids, metabolites, DNA and mRNA that are present in various organisms and cell types, both under normal conditions as well as during disease processes.

In recent years there have been significant advances to extend the emphasis of genome based studies from nucleotides to proteins and their functions, referred to as, "post-genomics" and/or "functional genomics;" of which proteomics is an integral part. The newly emerging field of proteomics comprises methodology to identify and characterize the function of specific proteins. Proteomics is the study of the proteome which is defined as the PROTEins expressed by a genOME (see for example : Proteome Research: New Frontiers in Functional Genomics", Wilkins, Williams, Appel, and Hochstrasser, Eds., 1997).

A variety of proteomics technologies are now being used to measure differences in cellular protein abundance. Currently, the primary method is two-dimensional (2D) sodium dodecylsulphate-polyacrylamide gel electrophoresis (SDS-PAGE) or chromatography coupled with mass spectrometry (MS). In this method, mixtures of proteins in cellular extracts are resolved and then individual proteins are identified, often using MS peptide fingerprinting (Pandey, A.; Mann, M. Nature 2000, 405, 837-846). Identification of proteins is just one small part of the field of proteomics. The specific function exerted by a protein is often altered by "post translational modification(s)" of a protein. The human genome project anticipated identifying over 100,000 genes from the human genome. However only 30,000-40,000 genes were identified and the most logical explanation for this apparent shortness in proteins is that post-translational modifications occur on over 80% of the proteins. This creates multiple forms of functional units from any one protein. Translation is a process whereby messenger RNA is converted to peptide chains. Enzymatic reactions occurring during and after the translation are called co- and post-translational modification. Modifications include phosphorylation, glycosylation, prenylation, demethylation, acetylation, myristoylation, palmitoylation, sulfation, etc. Predicted sites of protein modification can be determined using data bases such as EXPASY proteomic server (http://www.expasy.ch/www/tools). Most mammalian proteins (specifically cell surface and secreted molecules) are post-translationally modified. Besides proteins, also other type of molecules in the cell may be modified after completion of their biosynthesis. For example, lipids and anthyocyanins may be modified by addition of specific sugar structures (glycosylated). These modifications often serve multiple roles in conferring different structure and function to the same proteins, lipid or antocyanin under different conditions. Documenting the extent to which a cellular molecules are modified and the temporal changes in the modifications during disease can provide strategies for therapeutic intervention. Several approaches are being used to study post-translational modifications on a proteome-wide scale. Again, the most popular approach couples MS, which can detect even subtle covalent modifications, with methods to specifically enrich for modified proteins (Xhou, W., et al. J. Am. Soc. Mass Spectrom. 2000, 11, 273-282). Other strategies include the use of modification-specific antibodies (Srikrishna, G.; Wang, L.; Freeze, H. H. Glycobiol. 1998, 8, 799-811).

Proteomics technology offers a powerful tool to identify, in an unbiased approach, specific changes in protein profiles, related to development or to specific responses or conditions, such as (a-)biotic stress signals. However, proteomics technology is also considerably limited by the complexity of the full proteome of a cell. In addition, a total protein extract from a tissue typically consists of a combination of many different proteomes from the multiple different cell-types that may occur within a tissue. The complexity of a total protein extract from whole organisms or tissues may hinder the detection of subtle changes within protein profiles between samples on 2D-gel analysis because of limitations in resolution that can be obtained. As a result, many of the changes that may occur in specific cell types get potentially lost in the bulk of the unchanged protein pool that is extracted from a whole organism or whole tissue. 2D PAGE requires significant pre-separation processing of cell extracts, many of which must be optimized for the cell type being analyzed. Also, proteins present at low concentrations in the cell are generally not detected on the gels. Furthermore, each spot on a 2D gel is likely to represent more than one protein, since the 30,000+ proteins present in a cell at any given time cannot be completely resolved on a single gel. Perhaps most unacceptable, the reproducibility of 2D PAGE experiments is generally poor. This has prevented 2D gels from becoming the equivalent of a "gene chip" for protein profiling experiments, since it is difficult to compare data from multiple experiments.

Although in theory MS approaches have the potential to characterize the entire protein profile of a cell, in practice it has proved difficult to identify proteins of low abundance, because cell extracts, and the resulting mass spectra, are dominated by a few hundred very abundant proteins. For instance, analysis of the Arabidopsis seed proteome using 2D SDS-PAGE resulted in 1300 protein spots which could be resolved for further characterization (see for example Proteomic analysis of Arabidopsis seed germination and priming. Gallardo *et al.,* 2001, Plant Physiology 126, 835-848). However, only 78 out of the 1300 proteins were identified with significant changes in abundance during seed development.

Thus, although the technological revolution holds great promise to unravel the molecular profile or molecular repertoire of a cell or an organism, the field of molecular profiling calls for novel experimental approaches to reduce sample complexity and diversity and to increase detection sensitivity.

The invention provides a method for molecular profiling that allows for a more focussed analysis of molecular profiles and for detection of more subtle changes within molecular profiles. Provided is a method for identifying a molecular profile comprising a set of molecules of an organism, said method comprising the steps of:
a) providing an organism with at least one heterologous modifying activity capable of (covalent) tagging of at least part of said molecules with at least one tag which is otherwise not displayed in said organism, wherein said tagging is regulated by at least one regulatory element;
b) isolating the tagged molecules to obtain a library of tagged molecules; and
c) characterizing said tagged molecules.

A molecular profile refers to an essentially complete set of a certain type of biomolecules that is present in an organism or in a cell at a certain time or under certain conditions, be it specific or normal conditions. Biomolecules comprise a variety of molecules that are present or can be synthesized by a cell or an organism e.g. protein or polypeptide molecules, lipid molecules and metabolites such as flavonoids.

The term "heterologous modifying activity" as used herein refers to any enzymatic activity capable of providing a molecule that is present in an organism with a tag that is not displayed in said organism normally i.e. if said heterologous modifying activity is not present in said organism.

Thus, according to the invention various different modifying (enzyme) activities can be introduced into an organism to provide a subset of molecules of said organism with a heterologous tag followed by isolation and characterization of the tagged molecules. Two relevant criteria when deciding what type of modifying enzyme activity to introduce into an organism to practice a method according to the invention are: 1) the modifying activity should normally not be present in the organism and 2) the tag allows isolation of the tagged molecules to obtain a library of tagged molecules.

In one embodiment, the modifying activity is a post-translational modifying activity capable of providing a molecule, after its normal biosynthesis, with a tag that is not displayed in said organism normally. The term post-translational modification commonly refers to the modification of a protein after it has been translated. However, according to the present invention, post-translational modification (tagging) is not limited to proteins but also includes providing other biomolecules, such as lipids and flavonoids, following their biosynthesis with a tag. Many different post-translational activities have been described, including glycosylation, phosphorylation, acylation, acetylation, methylation, sulfation, farnesylation, myristoylation, prenylation, all of which may be used in a method as provided herein.

Methylation of proteins is one of the key reactions in the post-translational modification of protein amino acid residues in the cell. It has been established that the basic and acidic amino acid residues of certain proteins are methylated *in vivo.* Methylation can occur at lysine, arginine or carboxyl residues and is typically catalysed by a protein-specific methyltransferase (also referred to as a protein methylase) such as Protein methylase III (S-adenosylmethionine: proteinlysine methyltransferase; ED 2.1.1.25) and protein methylase I (S-adenosylmethionine:protein-arginine methyltransferase; EC 2.1.1.23). The activated methyl donor can be S-denosylmethionine.

Protein acylation is an important way in which a number of proteins with a variety of functions are modified. Several components of the protein acylation machinery in yeast have been characterized. These include myristoyltransferase, farnesyltransferase and palmitoyltransferase.

In a further embodiment, a heterologous modification comprises sulfate modification of sulfation. Sulfate modification of proteins occurs at tyrosine residues. Tyrosine O-sulfation is a common posttranslational modification of proteins in all multicellular organisms Tyrosine sulfation is a post-translational modification mediated by one of two Golgi isoenzymes, called tyrosylprotein sulfotransferases (TPST-1 and TPST-2). TPSTs catalyzes the transfer of sulfate from the universal sulfate donor 3'-phosphoadenosine 5'-phosphosulfate (PAPS) to the side-chain hydroxyl of lumenally oriented tyrosine residues within acidic motifs of proteins that transit the Golgi forming tyrosine 04-sulfate. A relatively small number of proteins are known to undergo tyrosine sulfation in man, including certain adhesion molecules, G-protein-coupled receptors, coagulation factors, serpins, extracellular matrix proteins, and hormones. Many of these proteins require tyrosine sulfation for optimal function. No plant TPST orthologs have yet been identified, and none is apparent in the completed *Arabidopsis thaliana* genome. Furthermore, no tyrosine-sulfated proteins, TPST activity, or putative TPSTorthologs have been described in prokaryotes or in yeast (see "The Biology and Enzymology of Protein Tyrosine *O*-Sulfation" K. L. Moore (2003) J. Biol. Chem., Vol. 278, 24243). According to a method of the invention, a sulfation activity is introduced in an organism which otherwise does not display said sulfation activity (e.g. plants) such that at least part of the biomolecules in said organism are provided with a sulfate tag. Of course, if the necessary precursor and/or substrate molecule(s) required for the heterologous sulfation are not present in said organism, they should be provided to the organism.

Furthermore, a modifying activity according to the invention can also be a co-translational modifying activity such as a protein glycosylation activity residing in the ER or a ubiquitination activity. In a preferred embodiment, an organism is provided with a heterologous glycosylation activity. Glycosylation refers to the adding of one or more oligosaccharide or carbohydrate residues to a molecule, such as a protein (polypeptide) or a lipid, in order to make a glycomolecule, such as a glycoprotein or glycolipid. Three different forms of protein glycosylation can be distinguished: N-linked oligosaccharides, O-linked oligosaccharides and glycosyl-phosphatidylinositol (GPI-) anchors. Early steps in protein glycosylation occur co-translational, subsequent modifications of the core glycan group occurs post-translational. early and late glycosylation occur in the endoplasmatic reticulum (ER) and the Golgi apparatus, respectively. Biogenesis of N-linked glycans [Schachter, 1991] begins with the synthesis of a lipid linked oligosaccharide moiety (Glc3Man9GlcNAc2-) which is transferred en bloc to the nascent polypeptide chain in the endoplasmatic reticulum (ER). Through a series of trimming reactions by exoglycosidases in the ER and cis-Golgi compartments, the so-called "high mannose" (Man9GlcNAc2 to Man5GlcNAc2) glycans are formed. Subsequently, the formation of complex type glycans starts with the transfer of the first GlcNAc onto Man5GlcNAc2 and further trimming by mannosidases to form GlcNAcMan3GlcNAc2. Complex glycan biosynthesis continues while the glycoprotein is progressing through the secretory pathway with the transfer in the Golgi apparatus of the second GlcNAc residue as well as other monosaccharide residues onto the GlcNAcMan3GlcNAc2 under the action of several glycosyl transferases. Plants and mammals differ with respect to the formation of complex glycans. In plants, complex glycans are characterized by the presence of b(1,2)-xylose residues linked to the Man-3 and/or an a(1,3)-fucose residue linked to GlcNAc-1, instead of an a(1,6)-fucose residue linked to the GlcNAc-1 [Lerouge et al., 1998]. Genes encoding the corresponding xylosyl and fucosyl transferases have been isolated. Plant members of the alpha1 -> ¾-fucosyltransferase gene family encode an alpha1-> 4-fucosyltransferase, potentially involved in Lewis(a) biosynthesis, and two core alpha1-> 3-fucosyltransferases. (Bakker et al. FEBS Lett. 2001 Nov 2;507(3):307-12.) Plants do not posses b(1,4)-galactosyltransferases (nor a(2,6)sialyltransferases (htti)://afmb.cnrs-mrs.fr/~cazy/CAZY/index.html (Coutinho, P.M. & Henrissat, B. (1999) Carbohydrate-active enzymes: an integrated database approach. In "Recent Advances in Carbohydrate Bioengineering", H.J. Gilbert, G. Davies, B. Henrissat and B. Svensson eds., The Royal Society of Chemistry, Cambridge, pp. 3-12; Coutinho, P.M. & Henrissat, B. (1999)) and consequently plant glycans lack the b(1,4)-galactose and terminal a(2,6)NeuAc residues often found on mammalian glycans (Vitale and Chrispeels, 1984; Lerouge et al., 1998]. The final glycan structures are not only determined by the mere presence of enzymes involved in their biosynthesis but to a large extend by the specific sequence of the various enzymatic reactions. The latter is controlled by discrete sequestering and relative position of these enzymes throughout the ER and Golgi (Rothman Nature 1992), which is mediated by the interaction of determinants of the transferase and specific characteristics of the sub-Golgi compartment for which the transferase is destined. A number of studies using hybrid molecules have identified that the transmembrane domains of several glycosyltransferases, including that of b(1,4)-galactosyltransferases, play a central role in their sub-Golgi sorting. Provided is a method for identifying a molecular profile comprising a set of molecules of an organism, said method comprising the steps of a) providing an organism with at least one heterologous glycosylation activity, allowing tagging of at least part of said molecules with at least one carbohydrate tag which is otherwise not displayed in said organism wherein said tagging is regulated by at least one regulatory element; b) isolating said tagged molecules to obtain a library of tagged molecules and c) characterizing said tagged molecules.

In one embodiment of the invention, a molecular profile refers to a protein profile, preferably a glycoprotein profile. As said, current methods used for analysing molecular profiles, e.g. those used in proteomics, are often not as sensitive or as specific as one would wish. For a large part, this is due to the complexity and diversity of the sample that is subjected to such an analysis, for instance a total cell or tissue extract. A method provided herein now allows for reducing the complexity of a molecular profile of an organism, by tagging at least part of the molecules within said molecular profile with a tag which is otherwise not present or displayed in an organism (e.g. a novel carbohydrate tag), removing or isolating the tagged molecules from the total sample and analysing only those tagged molecules. Provided that a molecule can be provided with a foreign molecular tag, a method according to the invention is also suitably used for identifying other molecular profiles other than protein profiles, such as flavonoid profiles, lipid profiles, and the like. In one embodiment, a method provided herein can for instance be used to reduce the complexity of a lipid profile of an organism by providing said organism with a heterologous modifying (such as glycosylation) activity that is capable of tagging one or more lipid molecules of said organism with a carbohydrate moiety to yield a tagged lipid that is normally not present in said organism. Likewise, other molecules or biomolecules susceptible for the introduced modifying activity (e.g. those comprising a glycosylation site such as flavonoids) can be tagged with a foreign or heterologous (glyco)tag in a regulated fashion.

The procedure to select a suitable heterologous modifying activity may comprise one or more of the following aspects:
· Inventory of the range of a specific type of modifying activity in an organism of interest (host) and of the resulting heterologous tag of molecules (e.g. glycosylation of proteins)
· Inventory of non-host specific modifying activities and resulting tagging of molecules (e.g. glycosylation of proteins) in organisms that may be mixed with host material (e.g. in host-pathogen interaction studies, see further below)
· Inventory of existing range of modifying activities in a host cell and of resulting tagging of molecules (e.g. glycosylation of proteins).
· Selection of non-host specific modifying activities not present in the material that needs to be analysed (e.g. host or host plus pathogen), using the following criteria:
· Selection of activity most suitable for the introduction and isolation of a (for host) novel modifying activity and resulting tag of molecules
· Assessment of presence of donor and acceptor substrate in host
· Selection of affinity molecule for purification of the tagged molecule available (e.g. lectin, antibody, Imab)
· Assurance of no/limited interaction of novel tagging activity with host biology
· Evaluate availability of the gene encoding the novel tagging activity in the host
· When required: adaptation of codon usage in novel tagging gene, to optimise for expression in host organism.

According to the invention, at least part of the molecules within the molecular profile of an organism is provided with a unique carbohydrate tag (also referred to as GlycoTag) that is normally not displayed in said organism, by introducing a heterologous glycosylation activity in said organism. A heterologous or 'foreign" glycosylation activity is a glycosylation activity which is otherwise not present in said organism. As said, differences in the specific glycosylation patterns among different species have been documented. For example, the glycosylation activities and herewith the type of glycosylation in mammals differs from that in insects, plants, yeast and fungi. A database of glycosylating activities in various organisms can for instance be found at http://afmb.cnrs-mrs.fr/~cazy/CAZY/index.html (Coutinho, P.M. & Henrissat, B. (1999) Carbohydrate-active enzymes: an integrated database approach. In "Recent Advances in Carbohydrate Bioengineering", H.J. Gilbert, G. Davies, B. Henrissat and B. Svensson eds., The Royal Society of Chemistry, Cambridge, pp. 3-12; Coutinho, P.M. & Henrissat, B. (1999) The modular structure of cellulases and other carbohydrate-active enzymes: an integrated database approach. In "Genetics, Biochemistry and Ecology of Cellulose Degradation"., K. Ohmiya, K. Hayashi, K. Sakka, Y. Kobayashi, S. Karita and T. Kimura eds., Uni Publishers Co., Tokyo, pp. 15-23.)

Provided is a method to identify a molecular profile, particularly a protein profile, of an organism wherein said organism is provided with a foreign or heterologous glycosylation activity, like for instance a (heterologous) enzyme catalysing O-linked or N-linked glycosylation. An organism is advantageously provided with said at least one heterologous glycosylation enzyme activity using at least one isolated or recombinant nucleotide sequence or gene encoding said activity. To control the tagging activity of the introduced novel enzyme activity, said sequence is preferably functionally coupled to said regulatory element. Of course, introducing a heterologous or foreign glycosylation activity in an organism should essentially be harmless for said organism e.g. it should not interfere with the endogenous metabolism of said organism. It has been shown that, upon the introduction of a heterologous glycosylation activity in an organism, said organism displays molecules with a unique carbohydrate tag. For example, the human Galactosyltransferase (GAlT) gene under the control of a constitutive promoter was introduced in tobacco plants, which are normally devoid of GalT activity (Bakker et al. 2001 PNAS Vol. 98 no.5 2899-2904). GalT transfers Gal from UDP-Gal in 1,4-linkage to GlcNAc residues in N-linked glycans of a glycoprotein. The transgenic plants showed no impairment of growth although protein analysis of the transformed plants revealed galactosylation of N-linked glycans.

As is exemplified herein, a heterologous tag (preferably a carbohydrate tag) is advantageously introduced into a cell or organism where it is otherwise not displayed to further reduce the complexity of a molecular (protein) profile. In addition, the invention provides the insight that heterologous tagging is advantageously extended to cell-type specific or developmentally tagging to provide a method for identifying spatially-restricted or time- molecular profiles. According to a method of the invention, the tagged molecules are sufficiently representative of the molecular profile of interest. A molecular profile may refer to a total set of a certain type of biomolecules but also to a specific subset of molecules e.g. molecules that are subject to a change upon exposure to a certain condition. In a preferred embodiment of the invention a substantial fraction or subset of the molecular profile, like more than 20%, better more than 30% , preferred over 40% or more preferred over 50% of the molecules is provided with a tag that is otherwise not displayed, such as a heterologous or foreign carbohydrate or glycotag. The fraction of the molecular profile provided with a molecular tag should be high enough to allow (following enrichment) the detection of the tagged molecules but low enough to maintain a normal phenotype of the organism. It may be advantageous to optimise the amount of heterologous modifying activity introduced into an organism (e.g. using promoters of different strengths) to optimize.
One can herein distinguish here between different sorts of subsets:
(1) Different percentage tagging of different potential target molecules that can by modified by the heterologous modifying activity (depending on molecule, this may vary from few percent to up to 50%)
(2) Different subset of molecules that are subject to a change upon introduction of a heterologous modifying activity in an organism :
   1. Subset determined by the extend/limitation of the promoter activity expressing the heterologous gene encoding the modifying activity
   2. Subset determined by the subcellular localisation of the heterologous modifying activity (eg cis-Golgi versus trans-Golgi)
   3. Subset determined by the substrate specificity of the heterologous modifying activity (eg 1-6 fucosyltransferase activity may tag a different set of proteins than the GalT activity).
   4. Combination of 1, 2 and / or 3.
For example, one can envision a situation wherein the expression of a subset of only 20 proteins out of a total of 10.000 proteins of an organism is changed, e.g. during a developmental process. This subset of 20 proteins is the molecular profile of interest. In a method according to the invention it is preferred that at least 4, or better more than 7, or even more than 10 of this subset of 20 proteins is provided with a molecular tag. Thus, a method provided is advantageously carried out with a heterologous modifying enzyme activity allowing tagging or modification of a substantive fraction of molecules within a given molecular profile. A particularly interesting carbohydrate tag for practicing a method as provided comprises a glycan-tag. It is estimated that about half of the proteome consists of glycosylated proteins, each of these proteins having specific patterns of either N- and/or O-linked glycosylation.

With respect to the modification of proteins by the addition of one or more carbohydrate residues, carbohydrate processing in the Golgi apparatus is called terminal or end-stage glycosylation to distinguish it from core glycosylation, which takes place in the ER. In a preferred embodiment, an organism is provided with a foreign or heterologous enzyme activity that catalyzes terminal glycosylation. The use of a heterologous, end-stage glycosylation activity in a method provided not only allows tagging of molecules with a tag that is otherwise not displayed in said organism. It also ensures that said tag is not further processed or modified and that it remains available for binding partners of the tag, so that it can be used to isolate and/or purify the tagged molecules. For instance, an organism that normally does not display terminal galactosylation, such as a plant or a plant cell, is provided with a galactosyltransferase (GalT) activity. Other suitable enzymes to effectuate (terminal) carbohydrate tagging comprise glucosyltransferase, fucosyltransferase and sialyltransferase.

In a preferred embodiment, a method provided involves providing a non-mammalian organism with a mammalian glycosylation activity to provide at least part of the molecules, preferably glycoproteins, of said non-mammalian organism with a mammalian carbohydrate tag. Said non-human organism is preferably selected from the group essentially consisting of a plant, a nematode, a fungus, and an insect.

For example, a plant cell is provided with a human glycosylation activity. In a further embodiment, the genome of an organism is essentially known because this will aid in selecting a foreign glycosylation activity. Of particular interest are the tobacco plant, the nematode *C. elegans* and the fly *D. melanogaster.*

In another aspect of the present invention, a host organism is a mutant or variant host organism with a disturbed or altered modifying activity when compared to the wild-type organism. The modifying activity in this mutant organism can be restored (e.g. in a cell type-specific manner) upon introduction of the normal (or wild-type) modifying activity in said mutant. For example, a mutant plant with disturbed glycosylation activity due to a mutation in an enzyme catalysing end-stage glycosylation is provided with a nucleic acid encoding the wild-type enzyme in a cell specific or organelle specific manner. As a result, only those cells or organelles provided with the end-stage glycosylation activity will molecules that are tagged with the particular glyco-tag. The tagged molecules can be easily isolated from the total pool of molecules present in the plant using a specific binding molecule, because the background of glyco-tagged molecules will be low.

In a method according to the invention, a heterologous modifying activity introduced into an organism is regulated by at least one regulatory element, such as a transcriptional promoter capable of regulating the modifying activity (tagging) introduced into specific cells or tissues of said organism. Whereas existing molecular profiling methods involved a mere reduction of the overall molecular complexity of a sample, it is now possible to spatially or temporally control the site and/or timing of the tagging (e.g. glycotagging) in an organism and thus to isolate and characterize a spatially addressable and/or temporally restricted set of molecules. For example, a heterologous or foreign glycosylation gene is introduced in an organism together with at least one regulatory element, preferably an element capable of driving expression of said glycosylation gene. In one embodiment, said at least one regulatory element comprises a spatially addressable or a restricted regulatory element, for example a tissue- or cell-type specific promoter.

In an other embodiment, said at least one regulatory element comprises a subcellular localization sequence (also known as targeting signal) to target a heterologous modifying activity to a subcellular compartment or organelle of interest to specifically tag a subset of molecules present at said subcellular compartment or organelle. To this end, a gene encoding a modifying activity can be fused to a subcellular localization sequence. Various subcellular sites have been identified which are suitably targeted used in a method according to the invention. These include targeting to the ER e.g. by the KDEL retrieval sequence or the targeting sequence of calreticulin, targeting to the Golgi apparatus using e.g. the targeting sequence from human beta 1; 4-galactosyltransferase, targeting to the peroxisomes by the peroxisomal targeting sequence PTS1, targeting to the plasma membrane using for instance the palmitoylation domain of neuromodulin or the farnesylation sequence of c-Ha-Ras, targeting to the mitochondria using the targeting sequence from subunit VIII of cytochropme c oxidase and nuclear targeting using e.g. the SV40 T-antigen nuclear localization signal (NLS). Herewith, the invention provides a method for identifying a molecular profile of an organism, wherein said molecular profile comprises a spatially addressable or a spatially restricted molecular profile. It is also possible to tag different subsets of proteins in the secretory pathways by providing a host cell with different glycosylating activities capable of tagging different subsets of molecules (see Review byBG Davis, Synthesis of Glycoproteins. Chem. Rev. 2002, 102,579-601)). In one embodiment, a mammalian GalT or a combination of mammalian GalT and GlnT is introduced in a plant. In another embodiment, 1-3FucT from a plant is introduced in a mammal, in a nematode or in Drosophila. In a specific embodiment, a heterologous modifying activity is introduced into a mammalian tumour cell which is subsequently transplanted into a mouse model. This allows to study a particular molecular profile of the tumour cell in the mouse. For example, a glycosylation activity of a plant is introduced into a tumor cell under the control of a drug-dependent promoter to specifically investigate alterations in the subset of molecules upon treatment with an (anti-cancer) drug. This may contribute to understand the mechanism of drug action.

In a preferred embodiment, a method is provided for identifying a protein profile wherein a unique carbohydrate or glycoside tag is introduced onto proteins present in only in those cells in which said regulatory element is present. A method provided allows to obtain a selection of proteins from a specific cell type in a total protein extract from tissue or whole organisms because a unique glycan-tag (GlycoTag) is added to proteins, more specifically glycoproteins, in just one cell-type of a multicellular organism or tissue. For example, a method provided now allows to decrease the complexity of the total glycoproteome of an organism, with the possibility to select the cell-type within a tissue or an organism from which the glycoproteome is analysed. Thus, the invention provides a method for molecular profiling with a cellular resolution and allows for (large scale) analysis of cell-type specific (glyco)proteomes of various organisms.

Other types of regulatory elements are however also advantageously used, including a temporally regulated promoter, to allow for the identification of a time-dependent molecular profile. In a preferred embodiment of the invention, an organism is provided with a foreign glycosylation activity under the control of a developmentally regulated promoter. For example, a set of plants is transformed with a foreign glycosylation activity, e.g. using the human galactosyltransferase gene, each under the control of a specific developmentally regulated promoter to selectively tag at least part of the molecules in a developmentally regulated fashion. Depending on the type of promoters used, isolation and characterization of the tagged molecules of each of the plants according to the invention can reveal several (developmental) molecular profiles such as those specific for root hairs, root meristem cells, apical meristem cells, trichomes , cells specifying specific floral whorls or floral organs, or phloem cells. In a method according to the invention for identifying a molecular profile of an organism, interesting regulatory elements are promoters that control a developmental program of said organism, i.e. those regulating the expression of one or more proteins involved in a signal transduction pathway, such as a pathway leading to proliferation, apoptosis or senescence. For example, in a method according to the invention a plant is provided with a foreign glycosylation activity that is under the control of a promoter of Nam, Clavata or MADsbox gene transcription.

A regulatory element according to the invention can also be an inducible promoter, preferably a promoter that is inducible by a hormone, light, a pathogen or stress. For instance, an organism such as a plant cell is transformed with a nucleic acid encoding a heterologous glycosylation activity under the control of a hormone- inducible promoter such as a promotor that is responsive to a plant hormone or phytohormone. Plant hormones include auxins, cytokinins, ethylene abscisic acid and gibberellins. Profiling the tagged proteins can reveal a specific receptor or other type of protein(s) involved in effectuating a phytohormone-specific response. Thus far, it has been difficult to detect subtle changes that may occur in the total protein profile of an organism upon exposure of said organism to an external or environmental factor. An advantage of a method provided by the invention is that a regulatory element which can be induced or regulated by an environmental or an external factor allows to selectively focus only on those set of molecules that are responsive to said factor. External factors are diverse, ranging from biotic/abiotic stress factors, such as host -pathogen interactions, to day-night rhythm.

In a method provided, an organism is provided with a foreign enzyme activity capable of catalysing the conversion of a molecule present in said organism into a molecule with a novel (carbohydrate) tag. It may however occur that not all the (carbohydrate) substrates required for said conversion are naturally present in said organism. Therefore, it is sometimes preferred to provide an organism not only with a foreign tagging activity but, if necessary, also with one or more molecules or substrates, or precursors thereof, that are required for said tagging. These 'foreign additives' are for instance simply added to the growth medium or food of said organism. In a preferred embodiment, a foreign additive is present continuously during the growth and propagation of said organism to ensure optimal (glyco)tagging of a set of molecules in a controlled fashion, e.g. cell specific-glycotagging of proteins. However, it is also possible to advantageously use a foreign additive as a regulatory element to control the timing of a foreign tagging activity in an organism. Provided that the tagging depends on the availability of a foreign additive, tagging can in theory only take place during the time period when the organism has access to the foreign additive. For instance, in one embodiment of the invention a plant is provided with a sialyltransferase activity that is normally not present in said plant. For sialylation to occur, the donor sugar nucleotide cytidine monophosphosialic acid (CMP-sialic acid) must be generated and enzymatically transferred to an acceptor oligosaccharide. Because CMP-sialic acid is normally not present in a plant, protein sialylation in a plant that is provided with a sialyltransferase activity only occurs when a (non-physiological) sialic acid precursor molecule is fed, such as CMP-sialic acid orUDP-siahc acid (E.G.T. Wee, D.J. Sherrier, T.A. Prime, D. Dupree. Targeting of active sialyltransferase to the plant Golgi apparatus, The Plant Cell 10, 1759-1768, 1998).

The choice of regulatory element to control the tagging of a (subset) of molecules essentially determines the focus of a selected group or library of molecules. For instance, because protein glycosylation takes place in the secretory pathway, a method provided particularly allows for uniquely tagging proteins of the secretory pathway by providing a host cell with a foreign glycosylation activity. In one aspect of the invention, a method is provided for identifying a molecular profile of secretory proteins, i.e. proteins that are or glycosylated or tagged in the secretory pathway.

The invention also provides use of a method to detect a change in the molecular profile of an organism. In one embodiment, an organism is provided with a glycosylation activity (that is normally not present in said organism) wherein said activity is controlled in a cell type-specific fashion. Subsequently, said organism is exposed to at least two different conditions, e.g. to a condition suspected to affect or influence the molecular profile of the specific cell-type and to a normal, control condition. Following isolation and characterization of the cell-specific tagged molecules, a comparison is made between the at least two molecular profiles to determine whether a change has occurred in the molecular profile as a consequence of exposure to the condition. In one embodiment, a developmentally regulated promoter is used to detect a developmental change. In another embodiment, use of a method is provided to detect a change in the molecular profile of an organism in response to an environmental change or an external signal, such as a change in the protein profile wherein said change is related to a host-pathogen interaction. An organism, like a fungus or a plant, is transformed with a foreign glycosylation activity together with a regulatory element responsive to an infection of said host organism to a pathogen. According to the invention, a molecular profile, or changes therein, of proteins secreted by said host cell during the infection can be identified.

In order to isolate or retrieve the tagged molecules from the bulk of molecules, it is preferred that a tagged molecule can be specifically recognized by and bound to a binding partner. In a method according to the invention, tagged molecules are isolated to obtain a library of tagged molecules using at least one specific binding partner of said at of least one tag, also referred to as 'epitope tag' or 'affinity tag'. One will understand that it is important, if not crucial, for practicing a method according to the invention, that a specific binding partner is only reactive with a molecule of an organism if it is provided with a foreign carbohydrate tag. Only then, it is possible to isolate and characterize a spatially restricted and/or temporally confined molecular profile. In one aspect of the present invention, a carbohydrate tag, for example a cell-type specific glycotag, is used to isolate and purify the tagged molecules using a tag-specific carbohydrate-binding molecule. A number of carbohydrate-binding partners have been reported that are suitably used in a method provided. For example, in one embodiment of the invention, an antibody specifically reactive with a carbohydrate tag, or a binding fragment equivalent thereto, is used to isolate tagged molecules, preferably glycoproteins from the total pool. Suitable antibodies include but are not limited to horseradish peroxidase antibodies that are suitable for the isolation of 1-3 xylosyl and 1-3 fucosyl tagged (plant) proteins). Tagged molecules (for instance with a carbohydrate tag or GlycoTag) can also be isolated using using specific lectins (e.g. RCA lectin, Vector Laboratories, USA) or an industrial molecular affinity body (iMab). Although functionally just as versatile as antibodies, an iMab has no homology with these class of compounds. iMab proteins have been optimized for industrial performance rather than a long term evolution to perform as part of the immune system in the bloodstream. The very low costs of iMabs forms a major breakthrough for processing industries; now it becomes possible that the most selective form of chromatography can be upscaled to the level of tons per minute.

In another embodiment, glyco-tagged molecules are isolated to obtain a library of carbohydrate tagged molecules using a lectin as binding partner of a carbohydrate tag. A lectin is a carbohydrate- or sugar-binding protein of non-immune origin that agglutinates cells or precipitates glycoconjugates. A lectin molecule typically contains at least two sugar-binding sites. Lectins occur in many types of organisms; they may be soluble or membrane-bound; they may be glycoproteins. The specificity of a lectin is usually defined by the monosaccharides or oligosaccharides that are best at inhibiting the agglutination or precipitation the lectin causes. Further general and specific information about lectins can be found at http://plab.ku.dk/tcbh/Lectins12/DiVirgilio/paper.htm or in Lectins, Biology, Biochemistry, Clinical Biochemistry, Volume 12, including Proceedings from the 17th International Lectin Meeting in Würzburg, 1997,edited by Edilbert van Driessche, Sonia Beeckmans and Thorkild C. Bøg-Hansen, published by TEXTOP, Lemchesvej 11, DK-2900 Hellerup, Denmark, ISBN number 87-984583-0-2.

In one embodiment, RCA-1 lectin (from *Ricin communis*) is used to isolate molecules tagged with a foreign or novel carbohydrate (glycan) tag, such as glycoproteins tagged with a terminal galactose residue (see for example Bakker et al. PNAS 2001, vol.98, no.5. 2899-2904). However, other carbohydrate-binding molecules can also be used in a method of the invention, including concavalin A and galectin. Concanavalin A is specific for high-mannose type N-glycans whereas galectins are specific for complex-type N-glycans with a terminal galactose.

To facilitate and speed up isolation of the tagged molecules, a binding partner (such as an antibody or other specific binding molecule or fragment) is preferably coupled to or immobilized onto a an insoluble matrix, such as a solid bead or a chromatography resin, to serve as affinity medium. In one aspect of the invention, a set of proteins tagged with a foreign glycan tag in a cell-type specific manner using a cell-specific promoter glycan is purified from the total protein pool using selective recapturing of the tagged glycomolecules by (repeating) lectin-column chromatography. Conjugation generally does not alter the specificity of the lectin.

Tagging of the molecules allows for a selective retrieval of the tagged molecules from the bulk of molecules, e.g. using affinity chromatography, to obtain a library of tagged molecules. Characterization of the tagged molecules can be performed using a variety of analytical techniques, including MS. Whereas it will of course depend on the type of heterologous modifying activity, the isolated subset of tagged molecules is generally much smaller than the total pool of molecules. Therefore, the tagging procedure greatly reduces the complexity of the molecular profile allowing for instance an easier characterization identification and/or more sensitive detection of differences between samples. Surprisingly, the simplified composition of a tagged protein library obtained according to the invention essentially makes the traditional 2D SDS-PAGE step essentially obsolete, because the protein extracts obtained through a method of the invention may be directly analysed using 2-dimensional liquid chromatography (2DLC)/MS or multidimensional liquid chromatography (MDLC)/MS technology.

The invention further provides a method to produce a library comprising a set of molecules of an organism wherein said molecules comprise a tag (e.g. a carbohydrate tag) which is otherwise not displayed in said organism, said method comprising the steps of providing an organism with at least one heterologous modifying (e.g. glycosylation) activity allowing tagging of said molecules with at least one tag which is otherwise not displayed in said organism, wherein said tagging is regulated by at least one regulatory element; and isolating the tagged molecules to obtain a library of tagged molecules.

Herewith, the invention provides a library comprising a set of molecules of an organism wherein said molecules comprise a tag which is otherwise not displayed in said organism. In a preferred embodiment, said library comprises tissue or cell-type specific tagged molecules. In another embodiment, a library comprises a set of tagged molecules of an organism that represents a time-dependent molecular profile. For instance, carbohydrate tagging in a plant is used in combination with a seed specific promoter to obtain libraries of glycoproteins during imbibition, priming and germination.
A library according to the invention is particularly useful for the identification of new molecular markers, such as protein markers and their encoding and regulatory nucleic acid sequences. For example, characterization of a library of the invention can reveal a set of proteins that is specifically expressed in a certain cell-type or under certain environmental conditions. This information is advantageously used to identify marker molecules for that cell-type or for that condition. In a further embodiment, the information obtained through a library of the invention is used to identify a molecular target for gene manipulation (RNAi). Other uses include but are not limited to the identification of a molecular target for diagnostic applications.

## Claims

1. A method for identifying a molecular profile comprising a set of molecules of an organism, said method comprising the steps of:
a) providing an organism with at least one heterologous modifying activity capable of tagging at least part of said molecules with at least one tag which is otherwise not displayed in said organism, wherein said tagging is regulated by at least one regulatory element;
b) isolating the tagged molecules to obtain a library of tagged molecules; and
c) characterizing said tagged molecules.

2. A method according to claim 1, wherein said modifying activity is selected from the group comprising glycosylation, , acylation, ubiquitiniation, sulfation, acetylation, methylation, prenylation, preferably glycosylation.

3. A method according to claim 1 or 2, wherein said molecular profile comprises a protein profile or a lipid profile.

4. A method according to claim 1-3, wherein said tagging comprises organelle, cell-type, or tissue-type specific tagging.

5. A method according to any one of claims 1-4, wherein said tagging comprises developmentally regulated tagging.

6. A method according to any one of claims 1-5, wherein said tagged molecules are isolated using at least one specific binding partner of said at least one tag.

7. A method according to claim 5 or 6, wherein said binding partner is an antibody or a binding fragment functionally equivalent thereto.

8. A method according to claim 6, wherein said binding partner is a lectin such as RCA lectin

9. A method according to any one of claims 1-8, wherein said tagging comprises end-stage or terminal glycosylation.

10. A method according to claim 9, wherein said modifying activity comprises a galactosyltransferase activity.

11. A method according to claim 10, wherein said modifying activity comprises a glucosyltransferase, fucosyltransferase, or sialyltransferase activity.

12. A method according to any one of claims 1-10, wherein said organism is a non-human organism, preferably selected from the group essentially consisting of a plant, a nematode, a fungus, and an insect.

13. A method according to any one of claims 1-12, wherein said regulatory element comprises a spatially addressable regulatory element, preferably a tissue- or cell-type specific promoter, or a developmentally regulated promoter.

14. A method according to any one of claims 1-13, wherein said regulatory element comprises an inducible promoter, preferably a promoter that is inducible by a hormone, light, a pathogen, stress or a drug.

15. Use of a method according to any one of claims 1-14 to detect a change in the molecular profile of an organism, wherein said change is preferably related to a developmental change, to an environmental change or a response to an external signal.

16. A method to produce a library comprising a set of molecules of an organism wherein said molecules comprise a tag, preferably a carbohydrate tag, which is otherwise not displayed in said organism, said method comprising the steps of:
a) providing an organism with at least one heterologous modifying activity allowing tagging of said molecules with at least one tag which is otherwise not displayed in said organism, wherein said tagging is regulated by at least one regulatory element;
b) isolating the tagged molecules to obtain a library of tagged molecules.

17. A library comprising a set of molecules of an organism wherein said molecules comprise a tag, preferably a carbohydrate tag, which is otherwise not displayed in said organism.

18. Use of a library according to claim 17 to identify a molecular target for gene manipulation or diagnostic application.
